(19) **Europäisches Patentamt / European Patent Office / Office européen des brevets**

(11) **EP 4 446 357 B1**

(12) **EUROPEAN PATENT SPECIFICATION**

(45) Date of publication and mention
of the grant of the patent:
**22.07.2026 Bulletin 2026/30**

(51) International Patent Classification (IPC):
**G16C 20/10** (2019.01)    **G16C 20/30** (2019.01)
**C08G 63/78** (2006.01)    **G06F 18/23** (2023.01)
**G06N 3/02** (2006.01)    **G16C 20/70** (2019.01)
**G16C 60/00** (2019.01)

(21) Application number: **23814346.5**

(22) Date of filing: **22.05.2023**

(52) Cooperative Patent Classification (CPC):
**G06N 3/048; C08G 63/78; G06F 18/23; G06N 3/08; G16C 20/10; G16C 20/30;** G16C 20/70; G16C 60/00

(86) International application number:
**PCT/JP2023/019013**

(87) International publication number:
**WO 2024/176478 (29.08.2024 Gazette 2024/35)**

(54) **METHOD FOR MAKING PREDICTIONS RELATED TO POLYCONDENSATION REACTION, INFORMATION PROCESSING DEVICE, AND PROGRAM**

VERFAHREN ZUR DURCHFÜHRUNG VON VORHERSAGEN IN BEZUG AUF POLYKONDENSATIONSREAKTIONEN, INFORMATIONSVERARBEITUNGSVORRICHTUNG UND PROGRAMM

PROCÉDÉ DE RÉALISATION DE PRÉDICTIONS RELATIVES À UNE RÉACTION DE POLYCONDENSATION, DISPOSITIF DE TRAITEMENT D'INFORMATIONS, ET PROGRAMME

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **24.02.2023 JP 2023027827**

(43) Date of publication of application:
**16.10.2024 Bulletin 2024/42**

(73) Proprietor: **DIC Corporation**
**Tokyo 174-8520 (JP)**

(72) Inventors:
• **NAGAO Atsushi**
**Takaishi-shi, Osaka 5920001 (JP)**
• **MATSUFUJI Yoshimasa**
**Takaishi-shi, Osaka 5920001 (JP)**
• **SHIBAMOTO Naoki**
**Takaishi-shi, Osaka 5920001 (JP)**
• **KARIURA Shinsuke**
**Tokyo, 1038233 (JP)**
• **BESSHO Nobuaki**
**Tokyo, 1038233 (JP)**

(74) Representative: **TBK**
**Bavariaring 4-6**
**80336 München (DE)**

(56) References cited:
**WO-A1-2022/163035**    **JP-A- 2022 149 072**

• **ABEYKOON CHAMIL ED - IEEE: "Design and Applications of Soft Sensors in Polymer Processing: A Review", IEEE SENSORS JOURNAL, IEEE, USA, vol. 19, no. 8, 15 April 2019 (2019-04-15), pages 2801 - 2813, XP011715326, ISSN: 1530-437X, [retrieved on 20190315], DOI: 10.1109/JSEN.2018.2885609**

- **YIN ZHANGQI ET AL: "Forecasting the Intrinsic Viscosity of Polyester Based on Improved Extreme Learning Machine", 2019 IEEE INTERNATIONAL CONFERENCE ON ARTIFICIAL INTELLIGENCE AND COMPUTER APPLICATIONS (ICAICA), IEEE, 29 March 2019 (2019-03-29), pages 241 - 246, XP033633381, DOI: 10.1109/ICAICA.2019.8873494**
- **JINTAO SHI ET AL: "Online prediction based on the Copula function for the intrinsic viscosity of polyester fibre", CANADIAN JOURNAL OF CHEMICAL ENGINEERING, WILEY SUBSCRIPTION SERVICES, INC., A WILEY COMPANY, HOBOKEN, USA, vol. 101, no. 3, 30 May 2022 (2022-05-30), pages 1440 - 1454, XP072541996, ISSN: 0008-4034, DOI: 10.1002/ CJCE.24431**
- **GENG JUNXIAN ET AL: "Fractal-based combined kernel function model for the polyester polymerization process", 2021 33RD CHINESE CONTROL AND DECISION CONFERENCE (CCDC), IEEE, 22 May 2021 (2021-05-22), pages 656 - 661, XP034032008, DOI: 10.1109/ CCDC52312.2021.9601586**
- **"Materials Informatics: A Super Introduction to Machine Learning for Materials Development, First Edition", 1 January 2019, NIKKAN KOGYO SHIMBUN, JP, ISBN: 978-4-526-07986-3, article YUUMA IWASAKI: "2.20 Data Preprocessing", pages: 52 - 54, XP009559444**

## Description

Technical Field

The present disclosure relates to a method for performing prediction related to a polycondensation reaction, an information processing device, and a program. The present application claims priority based on Japanese Patent Application No. 2023-027827 filed in Japan on February 24, 2023.

Background Art

Conventionally, methods for performing prediction related to chemical reactions have been developed (for example, PTL 1).

Citation List

Patent Literature

PTL 1: WO 2003/026791

WO 2022/163035 A1 discloses a polyester production system for producing a polyester through a reaction accompanied by changes in molecular weight. The system includes a memory unit for memorizing associative information which associates at least one piece of information on reaction conditions selected from among pieces of information on reaction conditions for the reaction, with molecular-weight information indicating molecular weights; an acquisition unit for acquiring at least one of the pieces of information on reaction conditions from a reactor in which the reaction is proceeding and for acquiring during-reaction molecular-weight information, which is information on the molecular weight at any point of time during the reaction; and a calculation unit for predicting the molecular weight of a reaction product being reacting, on the basis of said pieces of information on reaction conditions, said during-reaction molecular-weight information, and said associative information.

JP 2022 149072 A discloses a resin manufacturing process comprising a spectrum determination step in which the degree of progress of a polymerization reaction is determined by measuring the near-infrared absorption spectrum of a polymerization reaction mixture in the measurement wavenumber range of 9400 to 4500 cm-1, and a viscosity behavior determination step in which the degree of progress of the polymerization reaction is determined from the viscosity behavior of the polymerization reaction mixture. ABEYKOON CHAMIL ED - IEEE: "Design and Applications of Soft Sensors in Polymer Processing: A Review", IEEE SENSORS JOURNAL, IEEE, USA, vol. 19, no. 8, 15 April 2019 (2019-04-15), pages 2801-2813, ISSN: 1530-437X, DOI: 10.1109/JSEN.2018.2885609 discloses a comprehensive review on the use of soft sensing techniques in polymer processing applications while identifying their capabilities and limitations, and discusses the importance of developing of such soft sensing techniques (together with some sort of built-on intelligence) for the advancement of process monitoring, while indicating some of the possible directions for future industry.

YIN ZHANGQI ET AL: "Forecasting the Intrinsic Viscosity of Polyester Based on Improved Extreme Learning Machine", 2019 IEEE INTERNATIONAL CONFERENCE ON ARTIFICIAL INTELLIGENCE AND COMPUTER APPLICATIONS (ICAICA), IEEE, 29 March 2019 (2019-03-29), pages 241-246, DOI: 10.1109/ICAICA.2019.8873494, discloses a forecasting model of intrinsic viscosity of polyester based on an extreme learning machine.

JINTAO SHI ET AL: "Online prediction based on the Copula function for the intrinsic viscosity of polyester fibre", CANADIAN JOURNAL OF CHEMICAL ENGINEERING, WILEY SUBSCRIPTION SERVICES, INC., A WILEY COMPANY, HOBOKEN, USA, vol. 101, no. 3, 30 May 2022 (2022-05-30), pages 1440-1454, ISSN: 0008-4034, DOI: 10.1002/CJCE.24431, discloses introducing the Copula function into the modelling of the polymerization process.

GENG JUNXIAN ET AL: "Fractal-based combined kernel function model for the polyester polymerization process", 2021 33RD CHINESE CONTROL AND DECISION CONFERENCE (CCDC), IEEE, 22 May 2021 (2021-0522), pages 656-661, DOI:

10.1109/CCD052312.2021.9601586 introduces the fractal dimension feature selection method and combines the kernel function model based on STL decomposition (Seasonal-Trend decomposition procedure based on Loess) for the polyester polymerization process.

Summary of Invention

Technical Problem

The technique described in PTL 1 has described the use of modeling techniques such as neural networks, partial least squares, and principal component regression to optimize the control of reactor systems. However, specific design methods and optimization in performing predictions related to polycondensation reactions have not been considered, and there has been room for improvement in the prediction technology related to the polycondensation reactions.

An object of the present disclosure made in view of such circumstances is to improve the prediction technology related to the polycondensation reactions.

Solution to Problem

According to the present invention, a method for performing prediction related to an addition polymerization reaction, an information processing device, and a non-transitory computer-readable recording medium are provided as specified by the appended claims. The invention is set out in the independent claims. The dependent claims define advantageous embodiments. The embodiments that do not fall under the scope of the claims are to be interpreted as examples useful for understanding the disclosure.

Advantageous Effects of Invention

According to the method for performing prediction related to a polycondensation reaction, the information processing device, and the program of the present invention, the prediction technology related to the polycondensation reaction can be improved.

Brief Description of Drawings

FIG. 1 is a block diagram illustrating the schematic configuration of an information processing device according to the present embodiment.
FIG. 2 is a flowchart illustrating operations of the information processing device according to the present embodiment.
FIG. 3 is concept of a polycondensation reaction process according to the present embodiment.
FIG. 4A is a view illustrating the time transition of each piece of data pertaining to a dehydration temperature rising process.
FIG. 4B illustrates the time transition of categories related to the dehydration temperature rising process.
FIG. 5 illustrates one example of the accuracy verification result of a prediction model according to the present embodiment.
FIG. 6 illustrates one example of the accuracy verification result of the prediction model according to the present embodiment.
FIG. 7 is one example of a visualization graph according to the present embodiment.
FIG. 8 is one example of the conceptual view of a prediction model in the present embodiment.

Description of Embodiments

Hereinafter, a method for performing prediction related to a polycondensation reaction, an information processing device, and a program in an embodiment of the present disclosure will be described with reference to the drawings. A prediction target according to the embodiment of the present disclosure includes both batch reaction and continuous reaction. Examples of the main polymer materials synthesized by the polycondensation reaction according to the present embodiment include polyesters, polyamides, polyethylene terephthalate, urea resins, phenolic resins, silicone resins, alkyd resins, alkyd resin polyethers, polyglucosides, melamine resins, and polycarbonates. For example, the polycondensation reaction according to the present embodiment includes a dehydration-condensation reaction of a polyester.

In each of the drawings, the same symbols are assigned to identical or equivalent parts. In the description of the present embodiment, descriptions of the identical or equivalent parts are omitted or simplified as appropriate.

First, the overview of the present embodiment will be described. The method for performing prediction related to a polycondensation reaction in the present embodiment is executed by an information processing device 10. The information processing device 10 trains a prediction model based on actual data including a plurality of explanatory factors and an objective factor related to the polycondensation reaction. The information processing device 10 predicts the objective factor during the polycondensation reaction based on the explanatory factors related to the polycondensation reaction by the trained prediction model. The explanatory factors include a plurality of feature values obtained by clustering analysis of time-series data from a plurality of measurement instruments at a dehydration temperature rising process. The objective factor is characterized by including at least either a viscosity or an acid value.

As described above, according to the present embodiment, the explanatory factors include the feature values obtained by the clustering analysis of time-series data from the measurement instruments at the dehydration temperature rising process. The objective factor is characterized by including either a viscosity or an acid value. In the case where such an objective factor in the polycondensation reaction is predicted, the prediction accuracy can be improved by including, in the explanatory factors, the feature values obtained by the clustering analysis of the time-series data from the measurement instruments at the dehydration temperature rising process as described later. Therefore, according the present embodiment, the prediction technology related to the polycondensation reaction can be improved.

(Configuration of Information Processing Device)

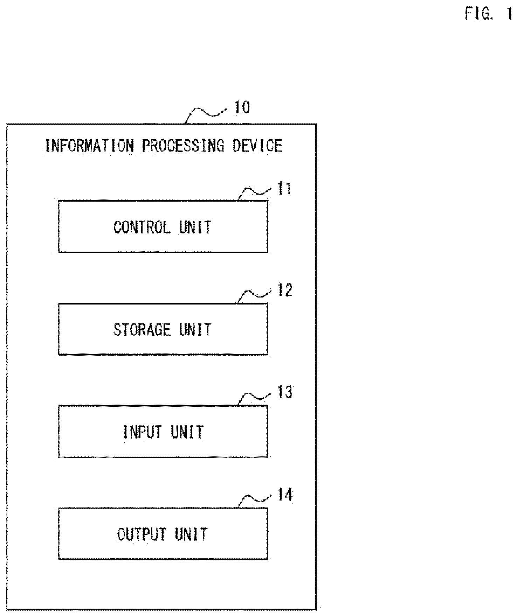

Subsequently, referring to FIG. 1, each configuration of the information processing device 10 will be described in detail. The information processing device 10 is an arbitrary device used by users. For example, personal computers, server computers, general-purpose electronic devices, or dedicated electronic devices can be employed as the information processing device 10.

As illustrated in FIG. 1, the information processing device 10 includes a control unit 11, a storage unit 12, an input unit 13, and an output unit 14.

The control unit 11 includes at least one processor, at least one dedicated circuit, or a combination thereof. The processor is a general-purpose processor such as a central processing unit (CPU) or a graphics processing unit (GPU), or a dedicated processor specialized for specific processing. The dedicated circuit is, for example, a field-programmable gate array (FPGA) or an application specific integrated circuit (ASIC). The control unit 11 executes processes associated with the operation of the information processing device 10 while controlling each part of the information processing device 10.

The storage unit 12 includes at least one semiconductor memory, at least one magnetic memory, at least one optical memory, or a combination of at least two of these memories. The semiconductor memory is, for example, a random-access memory (RAM) or a read-only memory (ROM). The RAM is, for example, a static random-access memory (SRAM) or a dynamic random-access memory (DRAM). The ROM is, for example, an electrically erasable programmable read-only memory (EEPROM). The storage unit 12 functions, for example, as a main memory device, an auxiliary memory device, or a cache memory. In the storage unit 12, data used in the operation of the information processing device 10 and data obtained by the operation of the information processing device 10 are stored.

The input unit 13 includes at least one interface for input. The interface for input is, for example, physical keys, capacitive keys, pointing devices, or touch screens integrated with displays. The interface for input may be, for example, a microphone that accepts voice input or a camera that accepts gesture input. The input unit 13 accepts operations to input data used in the operation of the information processing device 10. The input unit 13 may be connected to the information processing device 10 as an external input device instead of being provided in the information processing device 10. For example, any method such as universal serial bus (USB), high-definition multimedia interface (HDMI) (registered trademark), or Bluetooth (registered trademark) can be used as the connection method.

The output unit 14 includes at least one interface for output. The interface for output is, for example, a display that outputs information in the form of images. The display is, for example, a liquid crystal display (LCD) or an organic electroluminescence (EL) display. The output unit 14 displays and outputs data obtained by the operation of the information processing device 10. The output unit 14 may be connected to the information processing device 10 as an external output device instead of being provided in the information processing device 10. For example, any method such as USB, HDMI (registered trademark), or Bluetooth (registered trademark) can be used as the connection method.

The functions of the information processing device 10 are achieved by executing a program according to the present embodiment on a processor corresponding to the information processing device 10. In other words, the functions of the information processing device 10 are achieved by software. The program causes the computer to function as the information processing device 10 by causing the computer to execute the operations of the information processing device 10. In other words, the computer functions as the information processing device 10 by executing the operation of the information processing device 10 in accordance with the program.

In the present embodiment, the program can be recorded on computer-readable recording media. The computer-readable recording media include non-transient computer-readable media, for example, magnetic recording devices, optical discs, magneto-optical recording media, or semiconductor memories. Program distribution is performed by, for example, selling, assigning, or lending removal recording media such as a digital versatile disc (DVD) or a compact disc read only memory (CD-ROM) on which the program is recorded. Program distribution may also be done by storing the program in a storage on an external server and transmitting the program from the external server to other computers. The program may also be provided as a program product.

Some or all of the functions of the information processing device 10 may be achieved by a dedicated circuit corresponding to the control unit 11. In other words, some or all of the functions of the information processing device 10 may be achieved by hardware.

In the present embodiment, the storage unit 12 stores therein, for example, actual data and prediction models. The actual data and the prediction model may be stored in an external device separate from the information processing device 10. In this case, the information processing device 10 may be equipped with an interface for external communication. The interface for communication may be either an interface of a wired communication or an interface of wireless communication. In the case of the wired communication, the interface for communication is, for example, a LAN interface or USB. In the case of the wireless communication, the interface for communication is, for example, an interface compliant with mobile communication standards such as LTE, 4G, or 5G, or an interface compliant with short-range wireless communication such as Bluetooth (registered trademark). The interface for communication can receive data used in the operation of the information processing device 10 and can transmit data obtained by the operation of the information processing device 10.

(Operation of Information Processing Device)

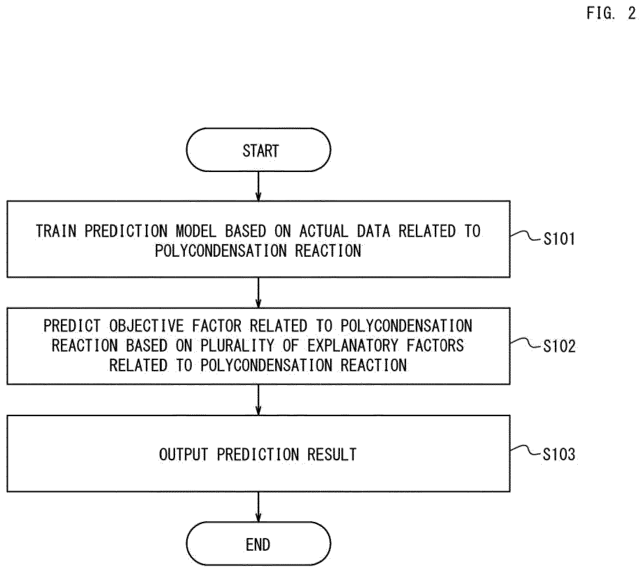

Subsequently, with reference to FIG. 2, the operation of the information processing device 10 according to the present embodiment will be described.

Step S101: The control unit 11 of the information processing device 10 trains a prediction model based on actual data related to the polycondensation reaction. The actual data include the explanatory factors and the objective factor related to the polycondensation reaction. The explanatory factors include the feature values obtained by the clustering analysis of the time-series data from the measurement instruments at the dehydration temperature rising process. The objective factor includes at least either a viscosity or an acid value. In other words, the control unit 11 trains the prediction model using these explanatory factors and objective factor included in the actual data as learning data.

Any method can be employed to acquire the actual data. For example, the control unit 11 acquires the actual data from the storage unit 12. The control unit 11 may also acquire the actual data by accepting input of the actual data from the user by the input unit 13. Alternatively, the control unit 11 may acquire such actual data from an external device that stores therein the actual data through an interface for communication.

The prediction model trained based on the learning data is cross-validated. As a result of such cross-validation, in the case where an accuracy is within a practical range, the prediction related to the polycondensation reaction is performed using the prediction model.

Step S102: The control unit 11 predicts the objective factor related to the polycondensation reaction based on the explanatory factors related to the polycondensation reaction. For example, the control unit 11 may acquire the explanatory factors by accepting input of the explanatory factors from the user by the input unit 13.

Step S103: The control unit 11 outputs the objective factor predicted at Step S102 as the prediction result from the output unit 14.

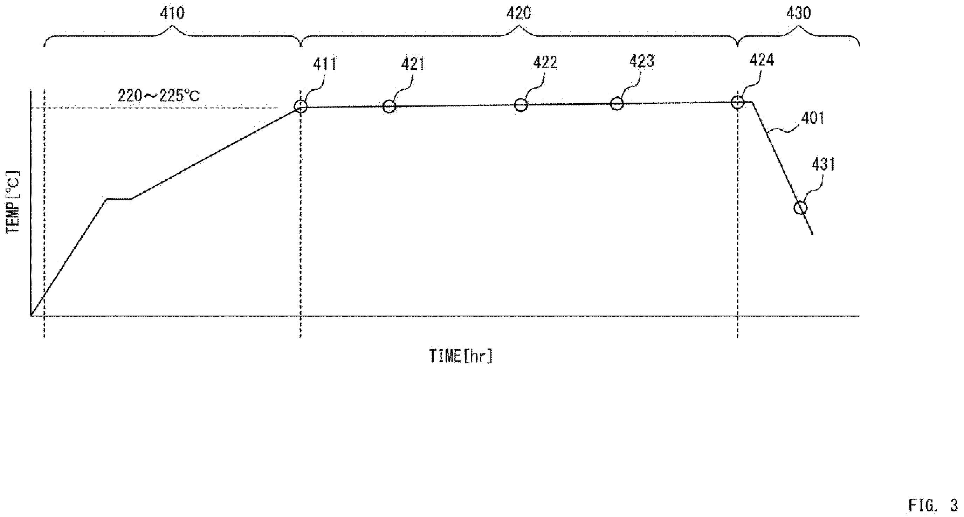

Here, in the present embodiment, the explanatory factors are characterized by including the feature values obtained by the clustering analysis of the time-series data from the measurement instruments at the dehydration temperature rising process. FIG. 3 is a conceptual view illustrating the polycondensation reaction process. As illustrated in FIG. 3, the polycondensation reaction includes a dehydration temperature rising process 410, a holding process 420, and a cooling process 430. A graph 401 illustrates the temperature transition of a material to be synthesized. At the dehydration temperature rising process 410, the temperature of the material to be synthesized rises. In the holding process 420, the temperature of the material to be synthesized is kept constant. At intermediate stages 421-424 of the holding process 420, the quality values of the material during the reaction are sampled and analyzed by hand. Such quality values include at least either a viscosity or an acid value. The quality values may include a hydroxyl value and physical property values related to color. Performing the sampling and the hand analysis at the intermediate stages 421-424 allows the time length of the holding process 420 to be adjusted. At a final stage 431 of the cooling process 430, the quality values of the material are analyzed.

The above analytical values in the polycondensation reaction correspond to the objective factor in the present embodiment. Such analytical values also depend on the dehydration temperature rising process. On the other hand, a wide variety of time-series data are involved at the dehydration temperature rising process, and thus using all of these time-series data as the explanatory variables is not realistic. Here, the time-series data include measurement values at intervals of 1 second to 1 minute. Therefore, the present embodiment is characterized by using the feature values obtained by the clustering analysis of the time-series data from the measurement instruments at the dehydration temperature rising process as the explanatory factors.

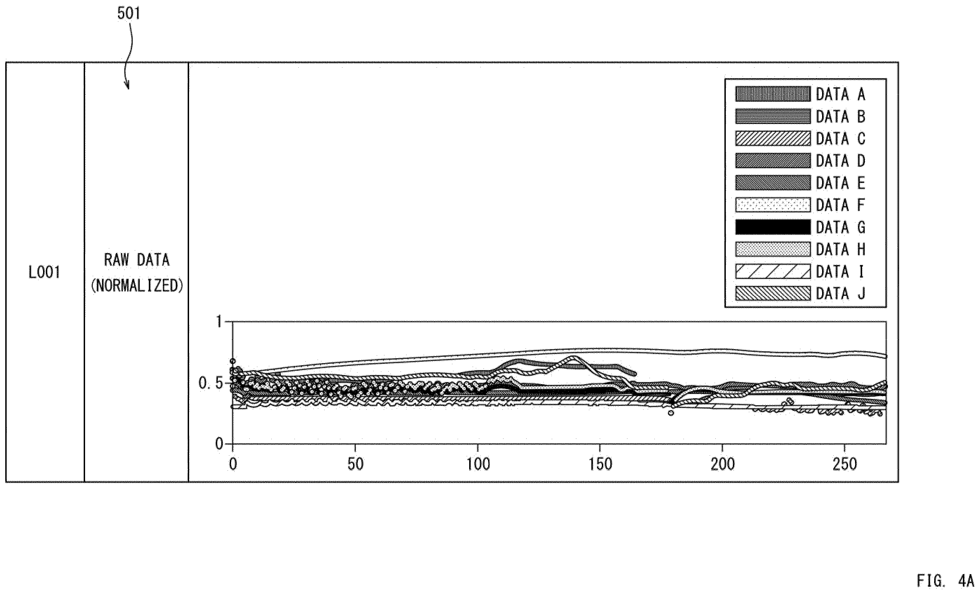

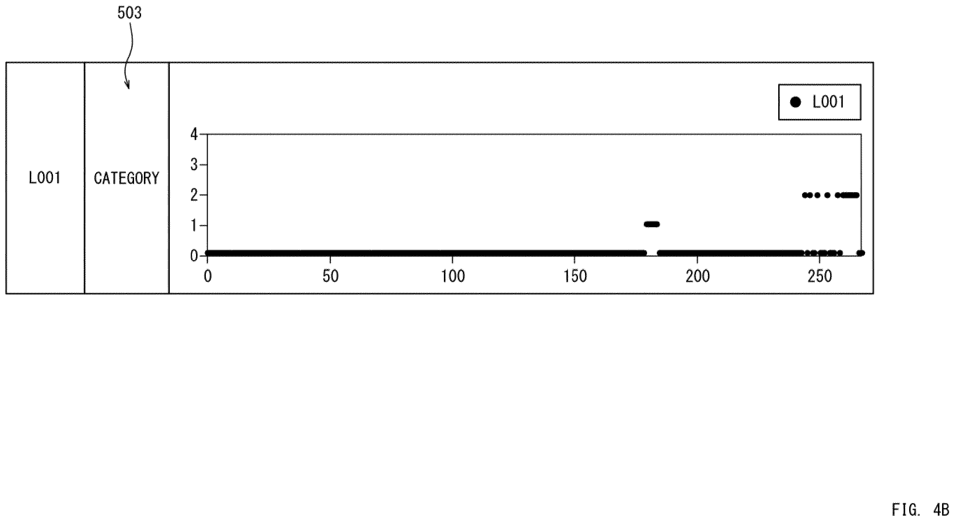

FIG. 4A and FIG. 4B illustrate a method for calculating the feature values in a certain lot (a lot having a lot number of L001). An item 501 in FIG. 4A represents the time transition of each piece of the data pertaining to the dehydration temperature rising process related to such a lot. Examples of such data include data pertaining to a vessel temperature, degasification, a column-top temperature, a reflux amount, a partial condenser inlet/outlet temperature, a heat medium, a vessel pressure, and a gas phase temperature. In FIG. 4A, the data pertaining to the dehydration temperature rising process are represented by Data A to Data J. Data A is data (°C/min) related to the gradient of the vessel temperature. Data B is data (kg/h/min) related to the gradient of a degassing volume. Data C is data (°C) related to the column-top temperature. Data D is data (kg/h/min) related to the gradient of the reflux amount (kg/h/min). Data E is data (°C) related to the inlet temperature to the partial condenser. Data F is data (°C) related to the outlet temperature from the partial condenser. Data G is data (°C/min) related to the gradient of the inlet temperature to the heat medium. Data H is data (°C/min) related to the gradient of the return temperature to the heat medium. Data I is data (MPa) related to the vessel pressure. Data J is data (°C/min) related to the gradient of the gas phase temperature. Each pieces of the data in the item 501 is normalized. An item 503 in FIG. 4B represents the time transition of the categories related to the dehydration temperature rising process. In the present embodiment, the categories include five steps of 0-4 . Specifically, the categories are determined by the clustering analysis of the time-series data from the measurement instruments at the dehydration temperature rising process. Based on these categories, the feature values in the dehydration temperature rising process are determined. According to the present invention, the feature values are determined based on the time rate of the categories. The time rate of the categories refers to a value obtained by dividing the cumulative residence time of each category by the overall time. As described above, in the present embodiment, the feature values are provided by the clustering analysis in the dehydration temperature rising process.

As described above, according to the present embodiment, the explanatory factors include the feature values obtained by the clustering analysis of the time-series data from the measurement instruments at the dehydration temperature rising process. The objective factor is characterized by including either a viscosity or an acid value. In the case where the prediction related to the polycondensation reaction is performed, the accuracy of the prediction model can be improved by including, in the explanatory factors, the feature values obtained by the clustering analysis of the dehydration temperature rising process. Therefore, according the present embodiment, the prediction technology related to the polycondensation reaction can be improved.

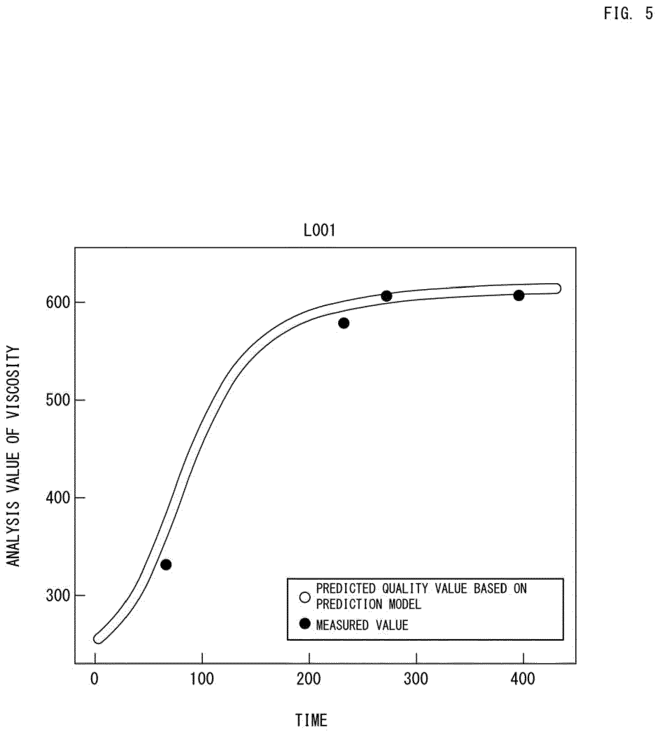

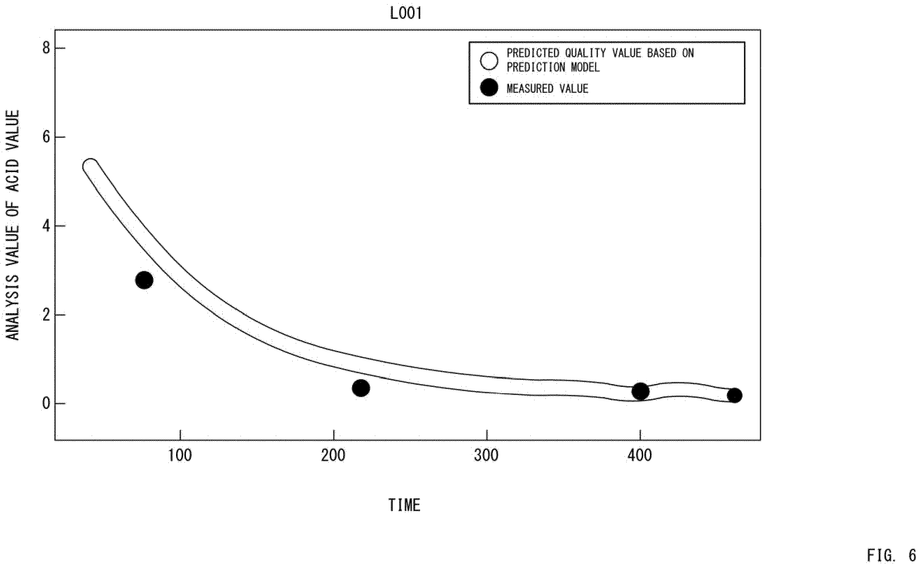

FIG. 5 and FIG. 6 illustrate an example of the accuracy verification results of the prediction model according to the present embodiment. FIG. 5 is a graph illustrating the viscosity of a certain lot (lot number L001) of polyester predicted by the prediction model and the measured values. As illustrated in FIG. 5, the viscosity predicted by the prediction model approximately agrees with the measured values. The prediction accuracy of the viscosity is ±1.85% relative to a standard value. FIG. 6 is a graph illustrating the acid value of the above lot of polyester predicted by the prediction model and the measured values. As illustrated in FIG. 6, the acid value predicted by the prediction model approximately agrees with the measured values. The prediction accuracy of the acid values is 0.085 in an absolute value relative to a standard value of 0.2 or less. Therefore, it is found that the accuracy of the prediction model is sufficiently high.

Here, the explanatory factors may include theoretical values in a reaction physics model. As described above, the reaction physics model may serve as explanatory factors as a baseline of the reaction characteristics. Similarly, the explanatory factors may include a heat medium return temperature, a vessel temperature, a nitrogen form, a yield, and a nitrogen amount.

In the present embodiment, a visualization graph may be output by previously predicting the change over time of the objective factor during the polycondensation reaction. Specifically, at the end point of the dehydration temperature rising process 410 (a dehydration temperature rising process end point 411), the continuous reaction progress to the reaction end point (before cooling) is previously predicted. Specifically, in this case, the explanatory factors include the cumulative calculation value of an added amount of a raw material (cumulative calculation value of raw material addition) and the raw material addition time during the polycondensation reaction. Then, the cumulative calculation value of raw material addition and the addition time thereof, which are some of conditions for calculating the predicted value in the change over time of the objective factor, are changed, whereby the visualization graph illustrating the relationship among a reaction end time, the amount of the added raw material, and the raw material addition time is output. Here, the reaction end time refers to a time until the physical property values of the material reach the target values. The amount of the added raw material refers to the added amount of the raw material that allows the product satisfying product specifications to be prepared in a single adjustment charge.

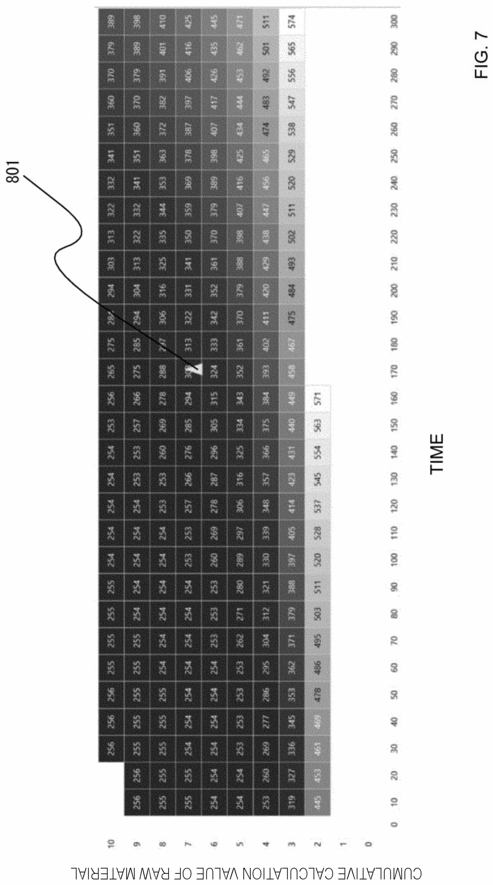

Such a visualization graph is an arbitrary graph in which a first axis is the raw material addition time and a second axis is the amount of the added raw material. For example, the visualization graph includes a heat map and a contour map. FIG. 7 illustrates one example of the visualization graph. FIG. 7 is one example in the case where the visualization graph is a heat map. In the heat map of FIG. 7, the horizontal axis corresponds to the first axis and represents the raw material addition time. The vertical axis of the heat map corresponds to the second axis and represents the amount of the added raw material. Each cell may indicate the numerical value of the reaction end time. In such a heat map, the shade of each cell changes depending on the reaction end time. Specifically, as the reaction end time becomes smaller, the shade of the cell becomes darker. This visualizes the relationship among the addition time of the added raw material, the amount of the added raw material, and the reaction end time. In other words, the addition time of the added raw material and the amount of the added raw material can be easily visually grasped in order to achieve the shortest possible reaction end time by the visualization graph such as the heat map.

Here, the visualization graph may include plots representing actual data. A plot 801 in FIG. 7 represents the actual data determining the added amount of the added raw material to be 7 and the addition time of the added raw material to be 170. The plot 801 of the actual data allows the optimal addition time and added amount of the added raw material to be determined by comparing and considering such actual data.

Here, the prediction model according to the present embodiment may be, for example, a neural network model. In the case where the prediction model is the neural network model, the coefficients of activation functions of the neural network model may be different between an intermediate layer and an output layer. For example, the coefficient of the activation function related to the intermediate layer is larger than the coefficient of the activation function related to the output layer.

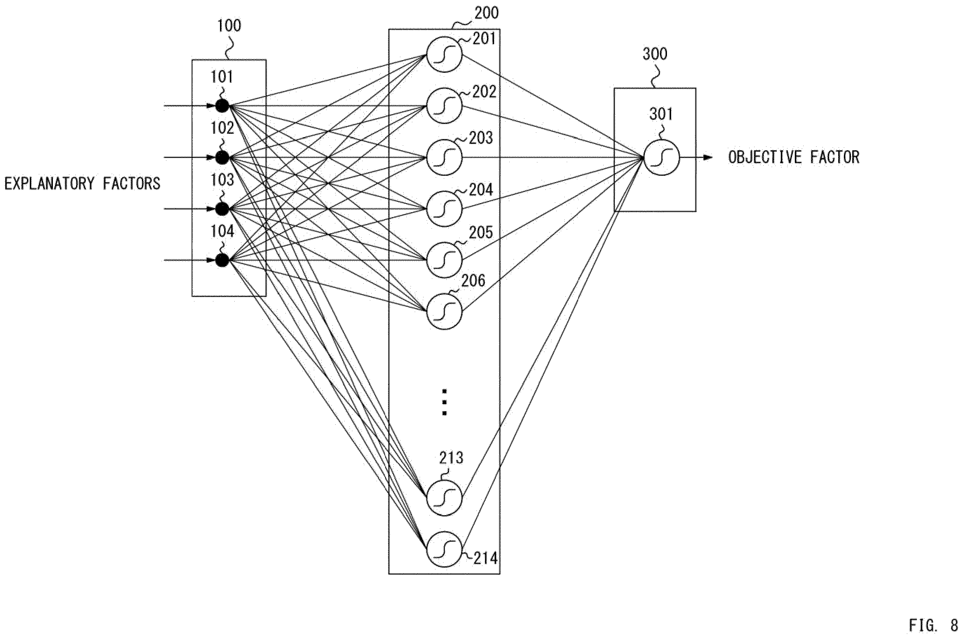

FIG. 8 is a conceptual view of the neural network model according to the present embodiment. Such a neural network model includes an input layer 100, an intermediate layer 200, and an output layer 300. The neural network model in the present embodiment is fully connected. In the present embodiment, the number of layers in the neural network model is, for example, 2. Such number of layers is the number of layers excluding the input layer. By setting the number of layers in the neural network model to 2, a model configuration can be prevented from becoming inappropriate to the physical phenomena in the polycondensation reaction. In other words, the number of layers of neural network model can be kept to minimum necessary, whereby the model configuration suitable for the physical phenomena in the polycondensation reaction can be achieved. The number of layers of the neural network model according to the present embodiment is not limited to this number of layers, and may be three layers or more. In the case where the number of layers in the neural network model is three or more, as the layer of the neural network model becomes more front side, the coefficient of the activation function may be set larger.

The input layer 100 includes a plurality of elements 101 to 104 (also referred to as input elements 101 to 104). In the neural network model illustrated in FIG. 8, the number of the input elements is 4. The input elements 101 to 104 are also referred to as the first to fourth elements, respectively. In the input elements 101 to 104, each of the explanatory factors is input. The number of input elements is not limited to this and may be less than 4 or 5 or more.

The intermediate layer 200 includes a plurality of elements 201 to 214 (also referred to as intermediate elements 201 to 214). In the neural network model illustrated in FIG. 8, the number of the intermediate elements is 14. The intermediate elements 201 to 214 are also referred to as the first to fourteenth elements, respectively. The number of intermediate elements is not limited to this, and may be less than 14 or 15 or more.

The output layer 300 includes an element 301 (an output element 301). In the neural network model illustrated in FIG. 8, the number of the output elements is 1. The output element 301 is also referred to as the first element The number of the output elements is not limited to this, and may be 2 or more.

The values input from the input elements 101 to 104 of the input layer 100 to the intermediate elements 201 to 214 of the intermediate layer 200 are converted in the intermediate layer 200 based on the activation function related to the intermediate layer 200. The converted values are output to the element 301 of the output layer 300. The activation function related to the intermediate layer 200 is, for example, a sigmoid function. The values input from the intermediate elements 201 to 214 of the intermediate layer 200 to the output element 301 of the output layer 300 are converted in the output layer 300 based on the activation function related to the output layer 300 and output. The activation function related to the output layer 300 is, for example, the sigmoid function. Specifically, the activation functions related to the intermediate layer and the output layer are, for example, the respective sigmoid functions determined by the following formulas (1) and (2).

[Mathematical Formula 1]

$$f^1\left(u_j^1\right) = \frac{1}{1+e^{-a_1 u_j^1}} \quad (1)$$

$$f^2\left(u_j^2\right) = \frac{1}{1+e^{-a_2 u_j^2}} \quad (2)$$

Here, $f^1\left(u_j^1\right)$ is the activation function related to the intermediate layer 200, $a_1$ is the coefficient of the activation function related to the intermediate layer 200, and $u_j^1$ is the input value input to the j-th element of the intermediate layer 200. In the example in FIG. 8, the number of the intermediate elements is 14, and this j takes the value from 1 to 14. $f^2\left(u_j^2\right)$ is the activation function related to the output layer 300, $a_2$ is the coefficient of the activation function related to the output layer 300, and $u_j^2$ is the input value input to the j-th element of the output layer 300. In the example in FIG. 8, j is 1 because the number of the output elements is 1. As described above, in the neural network model according to the present embodiment, the coefficient of the activation function related to the intermediate layer 200 is larger than the coefficient of the activation function related to the output layer 300. In other words, $a_1$ and $a_2$ in the neural network model according to the present embodiment satisfy $a_1 > a_2$.

In the neural network model according to the present embodiment, the coefficient of the activation function related to the intermediate layer is larger than the coefficient of the activation function related to the output layer. This allows the configuration of the neural network model to be optimized at the time of performing the prediction related to the polycondensation reaction. Specifically, in the neural network model for performing the prediction related to the polycondensation reaction, change in the explanatory factors is desirably viewed as obvious change. Therefore, by setting the coefficient of the activation functions related to the intermediate layer larger than the coefficient of the activation functions related to the output layer, the change in the input values to the intermediate layer can be transmitted to the output layer as the obvious change. On the other hand, in the output layer of the neural network model for performing the prediction related to the polycondensation reaction, the values of the training data and the objective factor are required to be converged. Therefore, the coefficient of the activation function related to the output layer is set smaller than the coefficient of the activation function related to the intermediate layer. By doing so, the value of the objective factor output from the output layer is finely adjusted.

By setting the coefficients of the activation functions between the intermediate layer and the output layer to be different, the learning process of the neural network model is optimized. Specifically, the updated amounts of the weight variables in the output layer and the intermediate layer during the learning process can be adjusted by changing the coefficient of the activation function. In addition, updating the weight variables provides a significant impact on the learning process. Therefore, the learning process may be optimized based on the adjustment of the updated amounts.

Specifically, in the neural network model at the time of performing prediction related to the polycondensation reaction, the updated amount of the weight variables related to the intermediate layer is preferably set to be relatively large. This allows the weight variables in the intermediate layer to vary more significantly during the learning process, and thus changes in the input values to the intermediate layer to be transferred to the output layer as obvious changes. On the other hand, the updated amount of weight variables related to the output layer are preferably set to be relatively small. This allows the weight variables in the output layer to vary less during the learning process and thus the values of the training data and the objective factor to be easily converged. In addition, by satisfying $a_1 > a_2$, an arbitrary smooth function can be approximated with sufficient accuracy, eliminating the need to inadvertently increase the number of intermediate layers. This allows sufficient accuracy to be obtained even when the intermediate layer is one layer. Preparing fewer intermediate layers directly leads to reduction in generation of over-fitting and thus provides a secondary effect on stability of the learning process and, in addition, robustness of the model.

The case where the activation functions of the intermediate layer and the output layer are sigmoid functions is described in the present embodiment. However, the activation functions are not limited to the sigmoid functions. For example, the activation functions of the intermediate layer and the output layer may be functions such as a hyperbolic tangent function (tanh function) and a ramp functions (ReLU).

Reference Signs List

| | |
|---|---|
| 10 | information processing device |
| 11 | control unit |
| 12 | storage unit |
| 13 | input unit |
| 14 | output unit |
| 100 | input layer |
| 200 | intermediate layer |
| 300 | output layer |
| 101 to 104, 201 to 214, and 301 | element |
| 401 | graph |
| 410 | dehydration temperature rising process |
| 411 | end point of dehydration temperature rising process |
| 420 | holding process |
| 421 to 424 | intermediate stage |
| 430 | cooling process |
| 431 | final stage |
| 501 and 503 | item |
| 801 | plot |

**Claims**

1. A method for performing prediction related to a polycondensation reaction executed by an information processing device (10), the method comprising:

a step of training (S101) a prediction model based on actual data comprising a plurality of explanatory factors and an objective factor related to the polycondensation reaction; and

a step of predicting (S102) the objective factor during the polycondensation reaction based on the explanatory factors related to the polycondensation reaction by the prediction model, wherein

the polycondensation reaction comprises a dehydration temperature rising process (410), a holding process (420), and a cooling process (430),

the explanatory factors include a plurality of feature values obtained by clustering analysis of normalized time-series data obtained by normalizing time-series data from a plurality of measurement instruments at the dehydration temperature rising process (410),

the time-series data from the measurement instruments at the dehydration temperature rising process comprises a plurality of data types out of the following types: a gradient of a vessel temperature, a gradient of a degassing volume, a column-top temperature, a gradient of a reflux amount, an inlet temperature to a partial condenser, an outlet temperature from the partial condenser, a gradient of an inlet temperature to a heat medium, a gradient of a return temperature to the heat medium, a vessel pressure, and a gradient of a gas phase temperature,

the clustering analysis comprises clustering the normalized time-series data comprising the plurality of data types in categories, and the feature values in the dehydration temperature rising process are determined based on a time rate of the categories,

the step of predicting predicts the objective factor during the holding process (420), and

the objective factor includes at least either a viscosity or an acid value, wherein

in the step of predicting, change over time of at least either the viscosity or the acid value during the holding process (420) is previously predicted, and

the explanatory factors include a cumulative calculation value of raw material addition and a raw material addition time during the polycondensation reaction, and

the method further changes the cumulative calculation value of raw material addition and the raw material addition time, which are some of conditions for calculating a predicted value in the change over time of the objective factor to output a visualization graph illustrating a relationship among a reaction end time, an amount of an added raw material, and the raw material addition time.

2. The method according to claim 1, wherein the explanatory factors include theoretical values in a reaction physics model.

3. The method according to claim 1, wherein the polycondensation reaction is a dehydration-condensation reaction of a polyester.

4. The method according to claim 1, wherein the visualization graph is a heat map or a contour map in which a first axis is the raw material addition time and a second axis is the amount of an added raw material.

5. The method according to claim 1, wherein the visualization graph includes plots representing actual data.

6. The method according to claim 1, wherein the prediction model is a neural network model including an input layer (100), an intermediate layer (200), and an output layer (300) and a coefficient of an activation function related to the intermediate layer is larger than a coefficient of an activation function related to the output layer.

7. An information processing device (10) performing prediction related to a polycondensation reaction, the information processing device (10) comprising a control unit (11), wherein
the control unit (10)

trains a prediction model based on actual data comprising a plurality of explanatory factors and an objective factor related to the polycondensation reaction, and

predicts the objective factor during the polycondensation reaction based on the explanatory factors related to the polycondensation reaction by the prediction model,

the polycondensation reaction comprises a dehydration temperature rising process (410), a holding process (420), and a cooling process (430),

the explanatory factors include a plurality of feature values obtained by clustering analysis of normalized time-series data obtained by normalizing time-series data from a plurality of measurement instruments at the dehydration temperature rising process,

the time-series data from the measurement instruments at the dehydration temperature rising process comprises a plurality of data types out of the following types: a gradient of a vessel temperature, a gradient of a degassing volume, a column-top temperature, a gradient of a reflux amount, an inlet temperature to a partial condenser, an outlet temperature from the partial condenser, a gradient of an inlet temperature to a heat medium, a gradient of a return temperature to the heat medium, a vessel pressure, and a gradient of a gas phase temperature, the clustering analysis comprises clustering the normalized time-series data comprising the plurality of data types in categories, and the feature values in the dehydration temperature rising process are determined based on a time rate of the categories, the control unit (10) predicts the objective factor during the holding process (420), and the objective factor includes at least either a viscosity or an acid value, wherein change over time of at least either the viscosity or the acid value during the holding process (420) is previously predicted, and the explanatory factors include a cumulative calculation value of raw material addition and a raw material addition time during the polycondensation reaction, and the cumulative calculation value of raw material addition and the raw material addition time, which are some of conditions for calculating a predicted value in the change over time of the objective factor, are changed to output a visualization graph illustrating a relationship among a reaction end time, an amount of an added raw material, and the raw material addition time.

8.  A non-transitory computer-readable recording medium storing therein instructions, in which when the instructions are executed by a processor of an information processing device (10) performing prediction related to a polycondensation reaction, the instructions cause the processor to execute:

    training of a prediction model based on actual data comprising a plurality of explanatory factors and an objective factor related to the polycondensation reaction; and
    prediction of the objective factor during the polycondensation reaction based on the explanatory factors related to the polycondensation reaction by the prediction model,

        the polycondensation reaction comprises a dehydration temperature rising process (410), a holding process (420), and a cooling process (430),
        the explanatory factors include a plurality of feature values obtained by clustering analysis of normalized time-series data obtained by normalizing time-series data from a plurality of measurement instruments at the dehydration temperature rising process,
        the time-series data from the measurement instruments at the dehydration temperature rising process comprises a plurality of data types out of the following types: a gradient of a vessel temperature, a gradient of a degassing volume, a column-top temperature, a gradient of a reflux amount, an inlet temperature to a partial condenser, an outlet temperature from the partial condenser, a gradient of an inlet temperature to a heat medium, a gradient of a return temperature to the heat medium, a vessel pressure, and a gradient of a gas phase temperature,
        the clustering analysis comprises clustering the normalized time-series data comprising the plurality of data types in categories, and the feature values in the dehydration temperature rising process are determined based on a time rate of the categories,
        the prediction comprises prediction of the objective factor during the holding process (420), and
        the objective factor includes at least either a viscosity or an acid value, wherein
        in the prediction, change over time of at least either the viscosity or the acid value during the polycondensation reaction is previously predicted, and
        the explanatory factors include a cumulative calculation value of raw material addition and a raw material addition time during the holding process (420), and
        the cumulative calculation value of raw material addition and the raw material addition time, which are some of conditions for calculating a predicted value in the change over time of the objective factor, are changed to output a visualization graph illustrating a relationship among a reaction end time, an amount of an added raw material, and the raw material addition time.

**Patentansprüche**

1.  Verfahren zum Durchführen einer Prädiktion hinsichtlich einer Polykondensationsreaktion, die durch eine Informationsverarbeitungseinrichtung (10) ausgeführt wird, wobei das Verfahren umfasst:

    einen Schritt eines Trainierens (S101) eines Prädiktionsmodells beruhend auf tatsächlichen Daten, die eine Vielzahl von erklärenden Faktoren und einen Zielfaktor hinsichtlich der Polykondensationsreaktion umfassen; und

einen Schritt eines Prognostizierens (S102) des Zielfaktors während der Polykondensationsreaktion beruhend auf den erläuternden Faktoren hinsichtlich der Polykondensationsreaktion durch das Prädiktionsmodell, wobei die Polykondensationsreaktion einen Dehydrationstemperaturanstiegsprozess (410), einen Halteprozess (420) und einen Kühlprozess (430) umfasst,

die erläuternden Faktoren eine Vielzahl von Merkmalswerten enthalten, die durch eine Clusteranalyse von normalisierten Zeitreihendaten erhalten werden, die durch Normalisieren von Zeitreihendaten von einer Vielzahl von Messinstrumenten bei dem Dehydrationstemperaturanstiegsprozess (410) erhalten werden,

die Zeitreihendaten von den Messinstrumenten bei dem Dehydrationstemperaturanstiegsprozess eine Vielzahl von Datentypen aus den folgenden Typen umfassen: Gradient einer Behältertemperatur, Gradient eines Ausgasungsvolumens, Säulenoberteiltemperatur, Gradient einer Rückflussmenge, Einlasstemperatur in einen Teilkondensator, Auslasstemperatur aus dem Teilkondensator, Gradient einer Einlasstemperatur in ein Wärmemedium, Gradient einer Rücklauftemperatur in das Wärmemedium, Behälterdruck und Gradient einer Gasphasentemperatur,

die Clusteranalyse ein Clustern der normalisierten Zeitreihendaten, die die Vielzahl von Datentypen umfassen, in Kategorien umfasst, und die Merkmalswerte in dem Dehydrationstemperaturanstiegsprozess beruhend auf einer Zeitrate der Kategorien bestimmt werden,

der Schritt des Prognostizierens den Zielfaktor während des Halteprozesses (420) prognostiziert, und

der Zielfaktor zumindest entweder eine Viskosität oder einen Säurewert enthält, wobei

in dem Schritt des Prognostizierens eine zeitliche Änderung zumindest entweder der Viskosität oder des Säurewerts während des Halteprozesses (420) vorab prognostiziert wird, und

die erläuternden Faktoren einen kumulativen Berechnungswert einer Rohmaterialzugabe und einer Rohmaterialzugabezeit während der Polykondensationsreaktion enthalten, und

das Verfahren ferner den kumulativen Berechnungswert der Rohmaterialzugabe und die Rohmaterialzugabezeit ändert, die einige von Bedingungen zur Berechnung eines prognostizierten Werts bei der zeitlichen Änderung des Zielfaktors sind, um ein Visualisierungsschaubild auszugeben, das eine Beziehung unter einer Reaktionsendezeit, einer Menge eines hinzugegebenen Rohmaterials und der Rohmaterialzugabezeit veranschaulicht.

2. Verfahren nach Anspruch 1, wobei die erläuternden Faktoren theoretische Werte in einem physikalischen Reaktionsmodell enthalten.

3. Verfahren nach Anspruch 1, wobei die Polykondensationsreaktion eine Dehydrationskondensationsreaktion von Polyester ist.

4. Verfahren nach Anspruch 1, wobei das Visualisierungsschaubild eine Heatmap oder eine Höhenlinienkarte ist, bei der eine erste Achse die Rohmaterialzugabezeit und eine zweite Achse die Menge an zugegebenem Rohmaterial sind.

5. Verfahren nach Anspruch 1, wobei das Visualisierungsschaubild Diagramme enthält, die tatsächliche Daten repräsentieren.

6. Verfahren nach Anspruch 1, wobei das Prädiktionsmodell ein neuronales Netzwerkmodell ist, das eine Eingabeschicht (100), eine Zwischenschicht (200) und eine Ausgabeschicht (300) enthält, und ein Koeffizient einer Aktivierungsfunktion hinsichtlich der Zwischenschicht größer als ein Koeffizient einer Aktivierungsfunktion hinsichtlich der Ausgabeschicht ist.

7. Informationsverarbeitungseinrichtung (10), die eine Prädiktion hinsichtlich einer Polykondensationsreaktion durchführt, wobei die Informationsverarbeitungseinrichtung (10) eine Steuereinheit (11) umfasst, wobei die Steuereinheit (10)

ein Prädiktionsmodell beruhend auf tatsächlichen Daten trainiert, die eine Vielzahl von erläuternden Faktoren und einen Zielfaktor hinsichtlich der Polykondensationsreaktion umfassen, und

den Zielfaktor während der Polykondensationsreaktion beruhend auf den erläuternden Faktoren hinsichtlich der Polykondensationsreaktion durch das Prädiktionsmodell prognostiziert,

die Polykondensationsreaktion einen Dehydrationstemperaturanstiegsprozess (410), einen Halteprozess (420) und einen Kühlprozess (430) umfasst,

die erläuternden Faktoren eine Vielzahl von Merkmalswerten enthalten, die durch eine Clusteranalyse normalisierter Zeitreihendaten erhalten werden, die durch Normalisieren von Zeitreihendaten von einer Vielzahl von Messinstrumenten bei dem Dehydrationstemperaturanstiegsprozess erhalten werden,

die Zeitreihendaten von den Messinstrumenten bei dem Dehydrationstemperaturanstiegsprozess eine Vielzahl von Datentypen aus den folgenden Typen umfassen: Gradient einer Behältertemperatur, Gradient eines Ausgasungsvolumens, Säulenoberseitentemperatur, Gradient einer Rückflussmenge, Einlasstemperatur in einen Teilkondensator, Auslasstemperatur aus dem Teilkondensator, Gradient einer Einlasstemperatur in ein Wärmemedium, Gradient einer Rückflusstemperatur in das Wärmemedium, Behälterdruck und Gradient einer Gasphasentemperatur,

die Clusteranalyse ein Clustern der normalisierten Zeitreihendaten, die die Vielzahl von Datentypen umfassen, in Kategorien umfasst, und die Merkmalswerte bei dem Dehydrationstemperaturanstiegsprozess beruhend auf einer Zeitrate der Kategorien bestimmt werden,

die Steuereinheit (10) den Zielfaktor während des Halteprozesses (420) prognostiziert, und

der Zielfaktor zumindest entweder eine Viskosität oder einen Säurewert enthält, wobei

eine zeitliche Änderung zumindest entweder der Viskosität oder des Säurewerts während des Halteprozesses (420) vorab prognostiziert wird, und

die erläuternden Faktoren einen kumulativen Berechnungswert einer Rohmaterialzugabe und eine Rohmaterialzugabezeit während der Polykondensationsreaktion enthalten, und

der kumulative Berechnungswert der Rohmaterialzugabe und die Rohmaterialzugabezeit, die einige von Bedingungen zur Berechnung eines prognostizierten Werts bei der zeitlichen Änderung des Zielfaktors sind, zur Ausgabe eines Visualisierungsschaubilds geändert werden, das eine Beziehung unter einer Reaktionsendezeit, einer Menge an zugegebenem Rohmaterial und der Rohmaterialzugabezeit veranschaulicht.

8. Nichtflüchtiges computerlesbares Aufzeichnungsmedium, das Instruktionen speichert, wobei, wenn die Instruktionen durch einen Prozessor einer Informationsverarbeitungseinrichtung (10) ausgeführt werden, die eine Prädiktion hinsichtlich einer Polykondensationsreaktion durchführt, die Instruktionen den Prozessor veranlassen, Folgendes auszuführen:

Trainieren eines Prädiktionsmodells beruhend auf tatsächlichen Daten, die eine Vielzahl von erläuternden Faktoren und einen Zielfaktor hinsichtlich der Polykondensationsreaktion umfassen; und

eine Prädiktion des Zielfaktors während der Polykondensationsreaktion beruhend auf den erläuternden Faktoren hinsichtlich der Polykondensationsreaktion durch das Prädiktionsmodell,

wobei die Polykondensationsreaktion einen Dehydrationstemperaturanstiegsprozess (410), einen Halteprozess (420) und einen Kühlprozess (430) umfasst,

wobei die erläuternden Faktoren eine Vielzahl von Merkmalswerten enthalten, die durch eine Clusteranalyse normalisierter Zeitreihendaten erhalten werden, die durch ein Normalisieren von Zeitreihendaten von einer Vielzahl von Messinstrumenten bei dem Dehydrationstemperaturanstiegsprozess erhalten werden,

wobei die Zeitreihendaten von den Messinstrumenten bei dem Dehydrationstemperaturanstiegsprozess eine Vielzahl von Datentypen aus den folgenden Typen umfassen: Gradient einer Behältertemperatur, Gradient eines Ausgasungsvolumens, Säulenoberseitentemperatur, Gradient einer Rückflussmenge, Einlasstemperatur in einen Teilkondensator, Auslasstemperatur aus dem Teilkondensator, Gradient einer Einlasstemperatur in ein Wärmemedium, Gradient einer Rückflusstemperatur in das Wärmemedium, Behälterdruck und Gradient einer Gasphasentemperatur,

wobei die Clusteranalyse ein Clustern der normalisierten Zeitreihendaten, die die Vielzahl von Datentypen umfassen, in Kategorien umfasst, und die Merkmalswerte bei dem Dehydrationstemperaturanstiegsprozess beruhend auf einer Zeitrate der Kategorien bestimmt werden,

wobei die Prädiktion eine Prädiktion des Zielfaktors während des Halteprozesses (420) umfasst, und

wobei der Zielfaktor zumindest entweder eine Viskosität oder einen Säurewert enthält, wobei

bei der Prädiktion eine zeitliche Änderung zumindest entweder der Viskosität oder des Säurewerts während der Polykondensationsreaktion vorab prognostiziert wird, und

die erläuternden Faktoren einen kumulativen Berechnungswert einer Rohmaterialzugabe und eine Rohmaterialzugabezeit während des Halteprozesses (420) enthalten, und

der kumulative Berechnungswert der Rohmaterialzugabe und die Rohmaterialzugabezeit, die einige von Bedingungen zur Berechnung eines prognostizierten Werts bei der zeitlichen Änderung des Zielfaktors sind, zur Ausgabe eines Visualisierungsschaubilds geändert werden, das eine Beziehung unter einer Reaktionsendezeit, einer Menge an zugegebenem Rohmaterial und der Rohmaterialzugabezeit veranschaulicht.

**Revendications**

1.  Procédé pour réaliser une prédiction liée à une réaction de polycondensation exécuté par un dispositif de traitement d'informations (10), le procédé comprenant :

    une étape d'apprentissage (S101) d'un modèle de prédiction basé sur des données réelles comprenant une pluralité de facteurs explicatifs et un facteur objectif liés à la réaction de polycondensation ; et
    une étape de prédiction (S102) du facteur objectif pendant la réaction de polycondensation basée sur les facteurs explicatifs liés à la réaction de polycondensation par le modèle de prédiction, où la réaction de polycondensation comprend un processus d'élévation de température de déshydratation (410), un processus de maintien (420), et un processus de refroidissement (430),
    les facteurs explicatifs comprennent une pluralité de valeurs de caractéristiques obtenues par analyse par regroupement de données de séries temporelles normalisées obtenues par normalisation de données de séries temporelles provenant d'une pluralité d'instruments de mesure lors du processus d'élévation de la température de déshydratation (410),
    les données de séries temporelles provenant des instruments de mesure lors du processus d'élévation de la température de déshydratation comprennent une pluralité de types de données parmi les types suivants : un gradient d'une température de cuve, un gradient d'un volume de dégazage, une température de tête de colonne, un gradient d'une quantité de reflux, une température d'entrée à un condenseur partiel, une température de sortie du condenseur partiel,
    un gradient d'une température d'entrée à un milieu thermique, un gradient d'une température de retour au milieu thermique, une pression de cuve, et un gradient d'une température de phase gazeuse,
    l'analyse par regroupement comprend le regroupement des données de séries temporelles normalisées comprenant la pluralité de types de données en catégories, et les valeurs de caractéristiques dans le processus d'élévation de la température de déshydratation sont déterminées sur la base d'un taux temporel des catégories,
    l'étape de prédiction prédit le facteur objectif pendant le processus de maintien (420), et le facteur objectif comprend au moins soit une viscosité, soit un indice d'acide, dans lequel dans l'étape de prédiction, l'évolution dans le temps d'au moins soit la viscosité, soit l'indice d'acide pendant le processus de maintien (420) est précédemment prédite, et
    les facteurs explicatifs comprennent une valeur de calcul cumulatif d'ajout de matière première et un temps d'ajout de matière première pendant la réaction de polycondensation, et
    le procédé modifie en outre la valeur de calcul cumulatif de l'ajout de matière première et le temps d'ajout de matière première, qui sont quelques-unes des conditions permettant de calculer une valeur prédite de l'évolution dans le temps du facteur objectif afin de produire un graphique de visualisation illustrant une relation entre un temps de fin de réaction, une quantité de matière première ajoutée, et le temps d'ajout de matière première.

2.  Procédé selon la revendication 1, dans lequel les facteurs explicatifs comprennent des valeurs théoriques dans un modèle de physique de réaction.

3.  Procédé selon la revendication 1, dans lequel la réaction de polycondensation est une réaction de déshydratation-condensation d'un polyester.

4.  Procédé selon la revendication 1, dans lequel le graphique de visualisation est une carte thermique ou une carte de contour dans laquelle un premier axe est le temps d'ajout de matière première et un deuxième axe est la quantité d'une matière première ajoutée.

5.  Procédé selon la revendication 1, dans lequel le graphique de visualisation comprend des tracés représentant des données réelles.

6.  Procédé selon la revendication 1, dans lequel le modèle de prédiction est un modèle de réseau neuronal comprenant une couche d'entrée (100), une couche intermédiaire (200), et une couche de sortie (300) et un coefficient d'une fonction d'activation liée à la couche intermédiaire est supérieur à un coefficient d'une fonction d'activation liée à la couche de sortie.

7.  Dispositif de traitement d'informations (10) réalisant une prédiction liée à une réaction de polycondensation, le dispositif de traitement d'informations (10) comprenant une unité de commande (11), dans lequel

    l'unité de commande (10)

entraîne un modèle de prédiction basé sur des données réelles comprenant une pluralité de facteurs explicatifs et un facteur objectif liés à la réaction de polycondensation, et

prédit le facteur objectif pendant la réaction de polycondensation basée sur les facteurs explicatifs liés à la réaction de polycondensation par le modèle de prédiction,

la réaction de polycondensation comprend un processus d'élévation de température de déshydratation (410), un processus de maintien (420), et un processus de refroidissement (430), les facteurs explicatifs comprennent une pluralité de valeurs de caractéristiques obtenues par analyse par regroupement de données de séries temporelles normalisées obtenues par normalisation de données de séries temporelles provenant d'une pluralité d'instruments de mesure lors du processus d'élévation de la température de déshydratation,

les données de séries temporelles provenant des instruments de mesure lors du processus d'élévation de la température de déshydratation comprennent une pluralité de types de données parmi les types suivants : un gradient d'une température de cuve, un gradient d'un volume de dégazage, une température de tête de colonne, un gradient d'une quantité de reflux, une température d'entrée à un condenseur partiel, une température de sortie du condenseur partiel, un gradient d'une température d'entrée à un milieu thermique, un gradient d'une température de retour au milieu thermique, une pression de cuve, et un gradient d'une température de phase gazeuse,

l'analyse par regroupement comprend le regroupement des données de séries temporelles normalisées comprenant la pluralité de types de données en catégories, et les valeurs de caractéristiques dans le processus d'élévation de la température de déshydratation sont déterminées sur la base d'un taux temporel des catégories,

l'unité de commande (10) prédit le facteur objectif pendant le processus de maintien (420), et le facteur objectif comprend au moins soit une viscosité, soit un indice d'acide, dans lequel l'évolution dans le temps d'au moins soit la viscosité, soit l'indice d'acide pendant le processus de maintien (420) est précédemment prédite, et

les facteurs explicatifs comprennent une valeur de calcul cumulatif d'ajout de matière première et un temps d'ajout de matière première pendant la réaction de polycondensation, et

la valeur de calcul cumulatif de l'ajout de matière première et le temps d'ajout de matière première, qui sont quelques-unes des conditions permettant de calculer une valeur prédite de l'évolution dans le temps du facteur objectif, sont modifiés afin de produire un graphique de visualisation illustrant une relation entre un temps de fin de réaction, une quantité de matière première ajoutée, et le temps d'ajout de matière première.

8. Support d'enregistrement non transitoire lisible par ordinateur stockant des instructions dans lequel lorsque les instructions sont exécutées par un processeur d'un dispositif de traitement d'informations (10) réalisant une prédiction liée à une réaction de polycondensation, les instructions amènent le processeur à exécuter :

un apprentissage d'un modèle de prédiction basé sur des données réelles comprenant une pluralité de facteurs explicatifs et un facteur objectif liés à la réaction de polycondensation ; et

une prédiction du facteur objectif pendant la réaction de polycondensation basée sur les facteurs explicatifs liés à la réaction de polycondensation par le modèle de prédiction,

la réaction de polycondensation comprend un processus d'élévation de température de déshydratation (410), un processus de maintien (420), et un processus de refroidissement (430),

les facteurs explicatifs comprennent une pluralité de valeurs de caractéristiques obtenues par analyse par regroupement de données de séries temporelles normalisées obtenues par normalisation de données de séries temporelles provenant d'une pluralité d'instruments de mesure lors du processus d'élévation de la température de déshydratation,

les données de séries temporelles provenant des instruments de mesure lors du processus d'élévation de la température de déshydratation comprennent une pluralité de types de données parmi les types suivants : un gradient d'une température de cuve, un gradient d'un volume de dégazage, une température de tête de colonne, un gradient d'une quantité de reflux, une température d'entrée à un condenseur partiel, une température de sortie du condenseur partiel,

un gradient d'une température d'entrée à un milieu thermique, un gradient d'une température de retour au milieu thermique, une pression de cuve, et un gradient d'une température de phase gazeuse,

l'analyse par regroupement comprend le regroupement des données de séries temporelles normalisées comprenant la pluralité de types de données en catégories, et les valeurs de caractéristiques dans le processus d'élévation de la température de déshydratation sont déterminées sur la base d'un taux temporel des catégories,

la prédiction comprend une prédiction du facteur objectif pendant le processus de maintien (420), et

le facteur objectif comprend au moins soit une viscosité, soit un indice d'acide, dans lequel dans la prédiction, une évolution dans le temps d'au moins soit la viscosité, soit l'indice d'acide pendant la réaction de polycondensation est précédemment prédite, et

les facteurs explicatifs comprennent une valeur de calcul cumulatif de l'ajout de matière première et un temps d'ajout de matière première pendant le processus de maintien (420), et

la valeur de calcul cumulatif de l'ajout de matière première et le temps d'ajout de matière première, qui sont quelques-unes des conditions permettant de calculer une valeur prédite de l'évolution dans le temps du facteur objectif, sont modifiés afin de produire un graphique de visualisation illustrant une relation entre un temps de fin de réaction, une quantité de matière première ajoutée, et le temps d'ajout de matière première.

FIG. 1

FIG. 2

```
                    ┌─────────────┐
                    │    START    │
                    └──────┬──────┘
                           │
                           ▼
┌──────────────────────────────────────────────────────┐
│  TRAIN PREDICTION MODEL BASED ON ACTUAL DATA RELATED TO│───S101
│              POLYCONDENSATION REACTION                 │
└──────────────────────────┬─────────────────────────────┘
                           │
                           ▼
┌──────────────────────────────────────────────────────┐
│  PREDICT OBJECTIVE FACTOR RELATED TO POLYCONDENSATION  │
│  REACTION BASED ON PLURALITY OF EXPLANATORY FACTORS    │───S102
│        RELATED TO POLYCONDENSATION REACTION            │
└──────────────────────────┬─────────────────────────────┘
                           │
                           ▼
┌──────────────────────────────────────────────────────┐
│              OUTPUT PREDICTION RESULT                  │───S103
└──────────────────────────┬─────────────────────────────┘
                           │
                           ▼
                    ┌─────────────┐
                    │     END     │
                    └─────────────┘
```

FIG. 3

EP 4 446 357 B1

501

| L001 | RAW DATA (NORMALIZED) | |

Legend:
- DATA A
- DATA B
- DATA C
- DATA D
- DATA E
- DATA F
- DATA G
- DATA H
- DATA I
- DATA J

FIG. 4A

503

| L001 | CATEGORY | |

FIG. 4B

FIG. 5

L001

FIG. 6

CUMULATIVE CALCULATION VALUE OF RAW MATERIAL

TIME

801

FIG. 7

FIG. 8

EP 4 446 357 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2023027827 A
- WO 2003026791 A
- WO 2022163035 A1
- JP 2022149072 A

### Non-patent literature cited in the description

- Design and Applications of Soft Sensors in Polymer Processing: A Review. IEEE SENSORS JOURNAL. IEEE, 15 April 2019, vol. 19, 2801-2813
- Forecasting the Intrinsic Viscosity of Polyester Based on Improved Extreme Learning Machine. **YIN ZHANGQI et al.** 2019 IEEE INTERNATIONAL CONFERENCE ON ARTIFICIAL INTELLIGENCE AND COMPUTER APPLICATIONS (ICAICA). IEEE, 29 March 2019, 241-246
- Online prediction based on the Copula function for the intrinsic viscosity of polyester fibre. **JINTAO SHI et al.** CANADIAN JOURNAL OF CHEMICAL ENGINEERING. WILEY SUBSCRIPTION SERVICES, INC., 30 May 2022, vol. 101, 1440-1454
- Fractal-based combined kernel function model for the polyester polymerization process. **GENG JUNXIAN et al.** 2021 33RD CHINESE CONTROL AND DECISION CONFERENCE (CCDC). IEEE, 22 May 2021, 656-661